(19) Europäisches Patentamt
European Patent Office
Office européen des brevets

(11) **EP 3 691 472 B1**

(12) **FASCICULE DE BREVET EUROPEEN**

(45) Date de publication et mention de la délivrance du brevet:
**16.11.2022 Bulletin 2022/46**

(21) Numéro de dépôt: **18792977.3**

(22) Date de dépôt: **01.10.2018**

(51) Classification Internationale des Brevets (IPC):
*A23L 11/30* (2016.01)   *A23J 1/14* (2006.01)
*A23J 3/14* (2006.01)   *A23L 33/185* (2016.01)

(52) Classification Coopérative des Brevets (CPC):
**A23J 3/14; A23J 1/14; A23L 11/30; A23L 33/185**

(86) Numéro de dépôt international:
**PCT/FR2018/052403**

(87) Numéro de publication internationale:
**WO 2019/068998 (11.04.2019 Gazette 2019/15)**

(54) **COMPOSITION DE PROTÉINES DE POIS A QUALITÉ NUTRITIONNELLE AMÉLIORÉE**

ERBSENPROTEINZUSAMMENSETZUNG MIT VERBESSERTEM NÄHRWERT

PEA PROTEIN COMPOSITION WITH IMPROVED NUTRITIONAL QUALITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **04.10.2017 FR 1759287**

(43) Date de publication de la demande:
**12.08.2020 Bulletin 2020/33**

(73) Titulaire: **Roquette Frères**
**62136 Lestrem (FR)**

(72) Inventeurs:
• **LECOCQ, Aline**
**59420 Mouvaux (FR)**
• **IBERT, Mathias**
**59930 La Chapelle D'armentieres (FR)**

(74) Mandataire: **Plasseraud IP**
**66, rue de la Chaussée d'Antin**
**75440 Paris Cedex 09 (FR)**

(56) Documents cités:
**WO-A1-2007/017572   WO-A1-2012/047252**

• **Anonyme: "Pea protein", Bulk Nutrients , 15 septembre 2015 (2015-09-15), XP002788006, Extrait de l'Internet: URL:https://www.bulknutrients.com.au/produ cts/pea-protein.html [extrait le 2019-01-16]**
• **REINKENSMEIER ANNIKA ET AL: "Characterization of individual proteins in pea protein isolates and air classified samples", FOOD RESEARCH INTERNATIONAL, ELSEVIER, AMSTERDAM, NL, vol. 76, 8 mai 2015 (2015-05-08), pages 160-167, XP029251386, ISSN: 0963-9969, DOI: 10.1016/J.FOODRES.2015.05.009**
• **Anonyme: "Roquette NUTRALYS® plant-based proteins: Trusted, Competitive, Unique!", Roquette , 25 janvier 2017 (2017-01-25), XP002788026, Extrait de l'Internet: URL:https://www.roquette.com/food-and-nutr ition/selected-ingredients/food-nutralys/ [extrait le 2019-01-16]**
• **Leterme P., Monmart T. and Baudart E.: "Amino acid composition of pea (Pisum sativum) proteins and protein profile of pea flour", Journal of the Science of Food and Agriculture, vol. 53 6 mai 1990 (1990-05-06), pages 107-110, XP002788010, Extrait de l'Internet: URL:https://www.researchgate.net/publicati on/230106119_Amino_acid_composition_of_pe a _Pisum_sativum_proteins_and_protein_profil e_of_pea_flour [extrait le 2019-01-16]**

EP 3 691 472 B1

**Description**

DOMAINE DE L'INVENTION

La présente invention a pour objet une composition de protéines de pois dont la qualité nutritionnelle, en particulier son PDCAAS, est améliorée, son procédé de préparation ainsi que l'utilisation de cette composition dans une composition alimentaire ou pharmaceutique.

ART ANTÉRIEUR

Les besoins quotidiens de protéines sont compris entre 12 et 20% de la ration alimentaire. Ces protéines sont fournies aussi bien par des produits d'origine animale (viandes, poissons, œufs, produits laitiers) que par des aliments végétaux (céréales, légumineuses, algues).

Cependant, dans les pays industrialisés, les apports en protéines sont majoritairement sous la forme de protéines d'origine animale. Or, de nombreuses études démontrent qu'une consommation excessive de protéines d'origine animale au détriment des protéines végétales est une des causes d'augmentation de cancers et maladies cardio-vasculaires.

Par ailleurs, les protéines animales présentent beaucoup de désavantages, tant sur le plan de leur allergénicité, concernant notamment les protéines issues du lait ou des œufs, que sur le plan environnemental en relation avec les méfaits de l'élevage intensif.

Ainsi, il existe une demande croissante des industriels pour des protéines d'origine végétale possédant des propriétés nutritionnelles et fonctionnelles intéressantes sans pour autant présenter les inconvénients des composés d'origine animale.

Depuis les années 70, le pois est la légumineuse à graines qui s'est le plus développée en Europe et majoritairement en France, notamment comme ressource protéique pour l'alimentation animale mais aussi humaine. Le pois contient environ 27 % en poids de protéines. Le terme « pois » est ici considéré dans son acception la plus large et inclut en particulier toutes les variétés sauvages de « pois lisse » (« smooth pea »), et toutes les variétés mutantes de « pois lisse » et de « pois ridé » (« wrinkled pea »), et ce quelles que soient les utilisations auxquelles on destine généralement lesdites variétés (alimentation humaine, nutrition animale et/ou autres utilisations).

La demande internationale WO 2012/047252 (D1) décrit qu'un isolat de protéines de pois a un taux de 90 à 95% de protéines totales par rapport au poids de l'extrait sec. Ce document rappelle également que les protéines solubles contenues dans le pois sont constituées de 65 à 80 % de globulines et de 20 à 35 % d'albumines.

Le document « Pea protein » (XP002788006, D2) décrit un isolat commercial de protéines de pois ayant une teneur protéique moyenne de 83% et pouvant être ajouté à une boisson.

Le document Food Research International. 2015, 76, 160-167 (D3) traite de la caractérisation des protéines dans des isolats de protéines de pois et farines de pois pouvant être utilisés pour la préparation de produits alimentaires.

Le document « Roquette Nutralys » (XP002788026, D4) décrit le produit Nutralys® commercialisé par la société demanderesse et pouvant être utilisé en nutrition ou alimentation spécialisée.

Le document J. Sci. Food Agric. 1990, 53, 107-110 (D5) traite de l'évaluation de l'impact d'un tamisage sur la composition protéique d'une farine de pois. Le document WO 2007/017572 (D6)décrit une composition de protéines de pois présentant une teneur en protéines sur matière sèche d'au moins 60 % en poids, de préférence comprise entre 60 et 95% en poids, et une teneur en facteurs anti-trypsiques comprise entre 2 et 5,5 UIT/mg.

Malgré leurs qualités indéniables, les protéines du pois souffrent d'un pouvoir nutritionnel en deçà des protéines animales et de soja. En effet, plusieurs études ont démontré que leur PDCAAS est inférieur à 1 (voir par exemple « Aliments à base de soja - source de protéines de haute qualité », ENSA, Aout 2015). Le PDCAAS, ou score en acides aminés corrigé de la digestibilité des protéines, sert à évaluer la qualité des protéines en fonction de deux critères : les besoins en acides aminés essentiels de l'homme et la digestibilité des protéines. Depuis 1989, l'OMS et la FAO conseillent d'utiliser cette méthode pour déterminer la qualité des protéines. Il est admis qu'une protéine parfaite d'un point de vue nutritionnel doit y obtenir un score de 1 (ou 100% selon l'expression du résultat).

De nombreux travaux et études ont essayé de pallier à cette lacune de la protéine de pois. Le PDCAAS inférieur à 1 est dû en partie au moins à la présence de facteurs antinutritionnels co-extraits avec la protéine de pois. On peut citer, par exemple les facteurs anti-trypsiques qui, en inhibant la trypsine digestive, perturbent la digestion de la protéine. La Demanderesse a donc développé un procédé de préparation d'une composition de protéines de pois comprenant majoritairement des globulines et ayant une faible teneur en facteurs antinutritionnels (voir WO2007017572). Le PDCAAS de cette composition est nettement amélioré en atteignant une valeur de 93% (ou 0,93) (voir « Vegetable Protein : A winner ? », C.Lefranc-Millot, 2014). Afin d'atteindre la valeur PDCAAS de 1, une solution bien connue consiste à ajouter à cette composition un mélange de protéines extraites du blé (voir toujours dans « Vegetable Protein : A winner ? », C.Lefranc-Millot, 2014). Cette solution élégante présente néanmoins plusieurs désavantages, tels que la nécessité d'enchainer plusieurs étapes afin d'obtenir la protéine désirée et l'utilisation de protéines de blé susceptibles de contenir des traces de gluten.

De plus, si l'on peut envisager l'obtention d'une protéine avec un PDCAAS de 1 par mélange de protéines autre que le pois, on obtient un mélange perdant les propriétés fonctionnelles de la dite protéine de pois, en particulier son pouvoir émulsifiant. La conservation d'un pouvoir émulsifiant, tout en augmentant son PDCAAS, afin de pouvoir facilement être formulées dans des recettes alimentaires est un requis majeur pour certaines applications industrielles, en particulier dans les domaines alimentaire, pharmaceutique et cosmétique.

Il existe donc toujours un besoin non satisfait d'une protéine extraite uniquement du pois dont la qualité nutritionnelle est équivalente à celles des protéines animales et dont les propriétés fonctionnelles, en particulier son activité émulsifiante, restent élevées

Il est du mérite de la Demanderesse d'avoir entrepris des travaux pour répondre à ces besoins et d'avoir élaboré la présente invention. En particulier, la Demanderesse a mis au point une composition ayant un PDCAAS de 1 comprenant uniquement des protéines extraites du pois en mélangeant la composition protéique de la demande WO2007017572, la fraction globuline du pois, avec un extrait de pois riche en albumines. Cette solution n'aurait pas été contemplée par l'homme du métier car les extraits de pois riches en albumines présentent également une forte teneur en facteurs anti-trypsiques. Les facteurs anti-trypsiques sont des albumines de faible poids moléculaire, de 8 à 10 kDa, qui sont capables de se lier de manière irréversible aux sites actifs de la trypsine. Ils empêchent ainsi l'hydrolyse gastro-intestinale des protéines ingérées par les protéases et diminuent donc la digestibilité des protéines et leur PDCAAS. En parvenant à abaisser la teneur en facteurs anti-trypsiques d'extraits de pois riches en albumines et en les combinant à des extraits de pois riches en globulines, la Demanderesse a pu obtenir une composition de protéines de pois ayant une excellente qualité nutritionnelle.

De plus, le mélange de la composition protéique de la demande WO2007017572, la fraction globuline du pois, avec un extrait de pois riche en albumines, est réalisée :

- En utilisant un extrait de pois riche en albumines particulier en ce que son activité émulsifiante est supérieur à 600 ml d'huile par gramme de protéines, préférentiellement supérieur à 800 ml d'huile par gramme de protéines, encore plus préférentiellement supérieur à 1000 ml d'huile par gramme de protéines.
- Et en réalisant ce mélange par voie humide puis en le séchant la solution ainsi obtenue, préférentiellement par atomisation.

## DESCRIPTION DÉTAILLÉE

Un premier objet de la présente invention est une composition de protéines de pois comprenant des globulines et des albumines caractérisée en ce que l'extrait sec de la composition :

- comprend au moins 80% en poids, de préférences de 80 à 99% en poids, plus préférentiellement de 85 à 98% en poids, de 90 à 97% en poids, de 92 à 95% en poids de protéines par rapport au poids de l'extrait sec ;
- présente un ratio massique des globulines sur les albumines de 65/35 à 85/15, de préférence de 70/30 à 82/18, plus préférentiellement de 75/25 à 80/20,

les albumines présentant une activité émulsifiante supérieure à 600 mL d'huile de maïs par gramme d'albumines, et l'extrait sec de la composition présente une teneur en facteurs anti-trypsiques de 1 à 5 UIT/mg.

De manière préférée, la composition est caractérisée par une activité émulsifiante supérieur à 300 ml d'huile par gramme de protéines, préférentiellement compris entre 300 et 500 ml d'huile par gramme de protéines, préférentiellement entre 350 et 450.

Un second objet de la présente invention est un procédé de préparation d'une composition de protéines de pois selon l'invention comprenant les étapes suivantes :

a) extraire les globulines et les albumines du pois pour obtenir une fraction protéique ;
b) séparer les globulines des albumines pour obtenir une fraction enrichie en globulines et une fraction enrichie en albumines ;
c) réduire la teneur en facteurs anti-trypsiques de la fraction enrichie en albumines pour obtenir une fraction enrichie en albumines traitée avec un procédé comprenant les étapes suivantes :

c-i) faire une microfiltration ou une centrifugation de la fraction enrichie en albumines pour obtenir un perméat de microfiltration ou un surnageant de centrifugation; et
c-ii) faire une ultrafiltration dudit perméat de microfiltration ou dudit surnageant de centrifugation pour obtenir un rétentat d'ultrafiltration, correspondant à la fraction enrichie en albumines traitée;

d) ajuster le pH à une valeur comprise entre 6,5 et 7,5 puis chauffer la fraction enrichie en albumines traitée à une température comprise entre 130°C et 150°C, préférentiellement 140°C, pendant une durée comprise entre 5 et 15 secondes, préférentiellement 10 secondes pour obtenir une fraction enrichie en albumines thermisée dont l'activité émulsifiante est supérieure à 600 ml d'huile par gramme de protéines, préférentiellement supérieure à 800 ml d'huile par gramme de protéines, encore plus préférentiellement supérieure à 1000 ml d'huile par gramme de protéines;

e) mélanger en présence d'eau la fraction enrichie en globulines et la fraction enrichie en albumines thermisée de manière à ce que l'extrait sec du mélange présente un ratio massique des globulines sur les albumines de 65/35 à 85/15, de préférence de 70/30 à 82/18, plus préférentiellement de 75/25 à 80/20.

f) sécher la solution ainsi obtenue.

De manière préférée, l'étape d) est réalisée en ajustant le pH entre 6 et 8, préférentiellement entre 6,5 et 7,5 et en chauffant entre 130°c et 150°C, préférentiellement de 140°C, avec un temps de traitement compris entre 5 et 15 secondes, préférentiellement de 10 secondes.

De manière préférée, l'étape f) est réalisée par atomisation, préférentiellement par atomisation dite « multiple-effet »

Un troisième objet de la présente invention est l'utilisation de la composition de protéines de pois selon l'invention dans une composition alimentaire ou pharmaceutique.

Le terme « pois » doit se comprendre dans la présente demande comme toutes les variétés sauvages de « pois lisse » (« smooth pea »), et toutes les variétés mutantes de « pois lisse » et de « pois ridé » (« wrinkled pea »).

Le terme « protéine » doit se comprendre dans la présente demande comme les macromolécules formées d'une ou de plusieurs chaînes polypeptidiques constituées de l'enchaînement de résidus d'acides aminés liés entre eux par des liaisons peptidiques. Dans le cadre particulier des protéines de pois, la présente invention concerne plus particulièrement les globulines (environ 50-60% en poids des protéines du pois) et les albumines (20-25% en poids des protéines du pois). Les globulines de pois se subdivisent principalement en trois sous-familles : les légumines, les vicilines et les convicilines. Les albumines de pois se subdivisent principalement en deux familles baptisées PA1 et PA2.

Le terme « facteurs anti-trypsiques » doit se comprendre dans la présente demande comme l'ensemble des composés possédant une activité inhibant les protéases digestives, en particulier la trypsine. La méthode de calcul de la teneur en facteurs anti-trypsique de la composition selon l'invention est détaillée dans les exemples de la présente demande.

Le terme « PDCAAS » doit s'entendre dans la présente invention comme le Protein Digestibility Corrected Amino Acid Scoring ou Score en Acides Aminés Corrigé de la Digestibilité des Protéines. Cette méthode est la plus utilisée aujourd'hui pour estimer la qualité protéique des aliments destinés à l'alimentation humaine. Le PDCAAS évalue la qualité des protéines en fonction de deux critères : les besoins en acides aminés essentiels de l'homme selon les recommandations de la FAO et la digestibilité des protéines. Depuis 1989, l'OMS et la FAO conseillent d'utiliser cette méthode pour déterminer la qualité des protéines. Il est admis qu'une protéine parfaite d'un point de vue nutritionnel doit y obtenir un score de 1 (ou 100% selon l'expression du résultat). La méthode de calcul du PDCAAS de la composition selon l'invention est détaillée dans les exemples de la présente demande.

Le terme « activité émulsifiante » doit se comprendre comme la quantité maximale d'huile pouvant être dispersée dans une solution aqueuse contenant une quantité définie d'émulsifiant avant rupture ou inversion de phase de l'émulsion (Sherman, P., 1995. A critique of some methods proposed for evaluating the emulsifying capacity and emulsion stabilizing performance of vegetable proteins. Ital. J. Food Sci., 1: 3.). Afin de la quantifier, la Demanderesse a développé un test permettant de la quantifier aisément, rapidement et de manière reproductible. Ce terme est également bien connu sous la terminologie « Concentration Micellaire Critique » ou « CMC ».

Les différents objets de l'invention seront mieux compris dans la description détaillée de l'invention qui suit.

La composition objet de la présente invention est une composition de protéines de pois qui comprend des globulines et des albumines.

Dans tout ce qui suit, les termes « protéines » « globulines » et « albumines » désignent respectivement des protéines, des globulines et des albumines extraites uniquement du pois. Ainsi, les protéines, globulines et albumines extraites d'une source végétale autre que le pois ou d'une source animale ne sont pas englobées par ces termes. Les globulines peuvent être distingués des albumines par diverses méthodes bien connues de l'homme du métier, notamment par leur solubilité dans l'eau, les albumines étant solubles dans l'eau pure alors que les globulines sont uniquement solubles dans l'eau salée. On peut également identifier les albumines et globulines présentes dans un mélange par électrophorèse ou chromatographie.

L'extrait sec de la composition selon l'invention comprend au moins 80% en poids, de préférences de 80 à 99% en poids, plus préférentiellement de 85 à 98% en poids, de 90 à 97% en poids, de 92 à 95% en poids, de protéines par rapport au poids de l'extrait sec. Toute méthode de dosage référence pour quantifier le taux de protéines bien connue de l'homme de l'art peut être utilisée. De préférence, on réalise un dosage de l'azote total (en %/brut) et on multiplie le résultat par le coefficient 6,25. Cette méthodologie bien connue dans le domaine des protéines végétales se base sur le constat que les protéines contiennent en moyenne 16% d'azote. Toute méthode de dosage de la matière sèche bien connue de l'homme du métier peut être également utilisée.

En outre, l'extrait sec de la composition selon l'invention présente un ratio massique des globulines sur les albumines de 65/35 à 85/15, de préférence de 70/30 à 82/18, plus préférentiellement de 75/25 à 80/20.

Selon un mode de réalisation particulier, les albumines contenues dans la composition selon l'invention consistent essentiellement en des albumines de type PA1 et PA2 et des lectines. Ainsi, le pourcentage en poids des facteurs anti-trypsiques par rapport au poids des protéines de la composition selon l'invention est inférieur au pourcentage en poids des facteurs anti-trypsiques par rapport au poids des protéines dans le pois à l'état naturel. De préférence, l'extrait sec de la composition selon l'invention présente une teneur en facteurs anti-trypsiques de 1 à 5 UIT/mg. La teneur en facteurs anti-trypsiques peut notamment être mesurée selon le procédé décrit ci-après.

Les albumines de la composition selon l'invention présentent une activité émulsifiante supérieure à 600, de préférence supérieure à 800, plus préférentiellement supérieure à 1000 mL d'huile de maïs par gramme d'albumines.

L'activité émulsifiante est définie comme la quantité maximale d'huile pouvant être dispersée dans une solution aqueuse contenant une quantité définie d'émulsifiant avant rupture ou inversion de phase de l'émulsion (Sherman, 1995). Afin de la quantifier, la Demanderesse a développé un test permettant de la quantifier aisément, rapidement et de manière reproductible. Ce procédé consiste à mettre en œuvre les étapes suivantes :

1. 0,2 g de l'échantillon du produit est dispersée dans 20 mL d'eau ;
2. la solution est homogénéisée avec un appareil Ultraturax IKA T25 pendant 30 sec à une vitesse de 9500 tours par minute (rpm) ;
3. ajout de 20 mL d'huile de maïs commercialisée sous la dénomination AMPHORA par la Société CARGILL sous homogénéisation dans les mêmes conditions que l'étape 2 précédente ;
4. centrifugation pendant 5 minutes à 3100 g ;

    a. si on obtient une bonne émulsion, on recommence le test à l'étape 1 en augmentant les quantités d'eau et d'huile de maïs de 50% ;
    b. si on obtient une mauvaise émulsion, par exemple un déphasage, on recommence le test à l'étape 1 en diminuant les quantités d'eau et d'huile de maïs de 50%.

La quantité maximale d'huile (Qmax en mL) pouvant être émulsifiée est ainsi déterminée de manière itérative.

L'activité émulsifiante est donc la quantité maximale d'huile de maïs pouvant être émulsifiée par grammes de produit.

$$\text{Activité émulsifiante} = (Q_{max} / 0,2)*100$$

Des albumines présentant une activité émulsifiante supérieure à 600 mL d'huile de maïs par gramme d'albumines peuvent notamment être obtenues par chauffage d'une fraction protéique comprenant des albumines tel que décrit dans l'étape d) du procédé ci-dessous.

Selon un mode de réalisation particulier, la composition selon l'invention présente un PDCAAS égal à 1 (ou 100% selon l'expression des résultats). En effet, la faible teneur en facteurs anti-trypsiques de la composition selon l'invention lui confère une bonne digestibilité. Le ratio massique des globulines sur les albumines dans la composition selon l'invention contribue en outre à un bon équilibre des acides aminés. La présence d'albumines permet notamment d'enrichir la composition en acides aminés soufrés. Ainsi, la composition selon l'invention présente une excellente qualité nutritionnelle.

La composition selon l'invention peut notamment être obtenue par le procédé de préparation d'une composition de protéines de pois décrit ci-après.

Le procédé de préparation d'une composition de protéines de pois objet de la présente l'invention comprend une étape a) dans laquelle les globulines et les albumines sont extraites du pois pour obtenir une fraction protéique.

Selon un mode de réalisation préférentiel, dans l'étape a) les globulines et les albumines sont extraites du pois avec un procédé comprenant les étapes suivantes :

a-i) broyer des pois ;
a-ii) introduire les pois broyés dans une solution aqueuse pour obtenir une phase solide A en suspension dans une phase liquide B ; et
a-iii) séparer la phase liquide B, correspondant à la fraction protéique, de la phase solide A.

Un tel procédé est notamment décrit dans la demande de brevet EP1400537.

Les pois mis en œuvre dans l'étape a-i) auront pu subir au préalable des étapes bien connues de l'homme du métier, telles que notamment un nettoyage (élimination des particules non désirées telles que pierres, insectes morts, résidus de terre, etc.) ou bien encore l'élimination des fibres externes du pois (enveloppe externe cellulosique) par une étape bien connue appelée « dehulling ».

Dans l'étape a-i), les pois peuvent être broyés en absence d'eau (procédé dit de « broyage à sec »). Selon un mode de réalisation alternatif, les pois peuvent être broyés en présence d'eau (procédé dit de « broyage humide »). Dans ce cas, l'étape a-ii) n'est pas mise en œuvre puisqu'on obtient directement une phase solide A en suspension dans une phase liquide B à l'issue de l'étape de broyage humide.

Dans l'étape a-ii), le pH de la solution aqueuse peut notamment être compris entre 6,2 et 7 et la température de la solution aqueuse peut notamment être comprise entre 5 et 20°C.

L'étape a-iii) permet de séparer la phase liquide B de la phase solide A. La phase liquide B correspond à la fraction protéique et est également appelée « fraction soluble ». Elle contient les protéines, en particulier les globulines et les albumines, ainsi que d'autres composés solubles dans la phase aqueuse, en particulier, des sels, des acides aminés et des glucides. La phase solide A contient quant à elle les fibres de pois et de l'amidon.

De préférence, la phase liquide B et la phase solide A sont séparées par fractionnement. La séparation par fractionnement peut notamment être réalisée au moyen de décanteurs centrifuges ou d'hydrocyclones.

Le procédé selon l'invention comprend une étape b) dans laquelle les globulines et les albumines sont séparées pour obtenir une fraction enrichie en globulines et une fraction enrichie en albumines.

Par « fraction enrichie en globulines » et « fraction enrichie en albumines », on entend une fraction ayant un pourcentage en poids de globulines, respectivement d'albumines, par rapport au poids des protéines dans ladite fraction qui est supérieur au pourcentage en poids de globulines, respectivement d'albumines, par rapport au poids des protéines dans le pois à l'état naturel. L'enrichissement correspond donc au pourcentage d'augmentation de la proportion de globulines, respectivement d'albumines, entre le pois à l'état naturel et la fraction enrichie. En particulier, l'enrichissement de la fraction enrichie est d'au moins 5%, 10%, 15%, 20%, 25%, 30%, 35%, 40%, 45%, ou 50% par rapport au pois à l'état naturel. L'enrichissement peut notamment être obtenu par purification et/ou concentration des fractions protéiques d'intérêt en globulines et en albumines. Ces fractions, selon le procédé appliqué, peuvent se présenter sous forme hydratée ou sèche.

L'étape b) peut notamment être mise en œuvre par précipitation des protéines à leur pH isoélectrique ou par séparation membranaire, par exemple par ultrafiltration.

Selon un mode de réalisation préférentiel, dans l'étape b) les globulines sont séparées des albumines avec un procédé comprenant les étapes suivantes :

b-i) faire floculer des globulines de la fraction protéique pour obtenir une phase solide C en suspension dans une phase liquide D ; et

b-ii) séparer la phase liquide D, contenant les albumines, de la phase solide C, contenant les globulines.

De préférence, dans l'étape b-i) la floculation des globulines est réalisée en amenant le pH de la fraction protéique au pH isoélectrique des globulines. Plus préférentiellement, le pH de la fraction protéique est ajusté à 4,5. La floculation des protéines peut notamment être réalisée en chauffant la fraction protéique à une température de 30 à 70°C, en particulier pendant 5 à 30 minutes, plus particulièrement entre 10 à 30 minutes.

Dans l'étape b-ii), la phase solide C (également appelée « floc ») est de préférence séparée de la phase liquide D par centrifugation. La phase solide C correspond à la fraction enrichie en globulines et comprend les globulines. La phase liquide D correspond à la fraction enrichie en albumines et comprend les albumines ainsi que d'autres composés solubles en phase aqueuse, en particulier, des sels, des acides aminés et des glucides.

Le procédé selon l'invention comprend une étape c) dans laquelle la teneur en facteurs anti-trypsiques de la fraction enrichie en albumines est réduite pour obtenir une fraction enrichie en albumines traitée.

Selon un mode de réalisation préférentiel, dans l'étape c) la fraction enrichie en albumines traitée présente une teneur en facteurs anti-trypsiques dans l'extrait sec de 20 à 80 UIT/mg, de préférence de 30 à 50 UIT/mg.

Dans l'étape c) la teneur en facteurs anti-trypsiques de la fraction enrichie en albumine est réduite avec un procédé comprenant les étapes suivantes :

c-i) faire une microfiltration ou une centrifugation de la fraction enrichie en albumines pour obtenir un perméat de microfiltration ou un surnageant de centrifugation; et

c-ii) faire une ultrafiltration dudit perméat de microfiltration ou dudit surnageant de centrifugation pour obtenir un rétentat d'ultrafiltration, correspondant à la fraction enrichie en albumines traitée.

La microfiltration de l'étape c-i) conduit à un perméat et à un rétentat de microfiltration. C'est le perméat qui est ensuite traitée dans l'étape ultérieure c-ii) d'ultrafiltration. La centrifugation de l'étape c-i) conduit quant-à-elle à un surnageant et à un sédiment de centrifugation. C'est le surnageant qui est ensuite traité dans l'étape ultérieure c-ii) d'ultrafiltration.

Lorsque l'étape c-i) est une microfiltration, celle-ci est préférentiellement une microfiltration tangentielle membranaire. Plus particulièrement, la microfiltration tangentielle est préférentiellement réalisée avec une membrane céramique présentant une porosité de 0,01 $\mu$m à 1 $\mu$m, préférentiellement de 0,05 $\mu$m à 0,5 $\mu$m.

L'étape c-ii) d'ultrafiltration est effectuée sur le perméat de microfiltration ou sur le surnageant de centrifugation. Elle permet d'obtenir d'une part un rétentat d'ultrafiltration riche en albumines, et d'autre part un perméat riche en sels, en acides aminées et en glucides. Plus particulièrement, il est recommandé de réaliser l'ultrafiltration à l'aide d'une membrane présentant un seuil de coupure compris entre 0.1 et 0.5 $\mu$m, la pression transmembranaire étant maintenue inférieure à 4 bars.

Le procédé selon l'invention comprend une étape d) dans laquelle la fraction enrichie en albumines traitée est soumise à un ajustement de pH puis à un chauffage pour obtenir une fraction enrichie en albumines thermisée. Cette étape permet notamment d'obtenir des albumines présentant une activité émulsifiante supérieure à 600 mL d'huile de maïs par gramme d'albumines.

Dans l'étape d) le pH de la fraction enrichie en albumines traitée est ajusté à une valeur comprise entre 6 et 8, de préférence entre 6,5 et 7,5. Le pH de la fraction enrichie en albumines traitée peut notamment être ajusté par ajout d'une base choisie parmi la soude, la potasse ou l'ammoniaque, préférentiellement de la soude. A noter que l'utilisation du carbonate est à éviter car il a une action néfaste sur le goût de la fraction albumine obtenue.

Le chauffage de la fraction enrichie en albumines traitée après l'ajustement de pH est un chauffage de type UHT, c'est-à-dire un chauffage à une température très élevée pendant un temps court. Dans l'étape d) la fraction enrichie en albumines traitée est chauffée à une température comprise entre 130°C et 150°C, préférentiellement 140°C, pendant une durée comprise entre 5 et 15 secondes, préférentiellement 10 secondes.

Le procédé selon l'invention comprend une étape e) dans laquelle la fraction enrichie en globulines et la fraction enrichie en albumines thermisée sont mélangées de manière à ce que l'extrait sec du mélange présente un ratio massique des globulines sur les albumines de 65/35 à 85/15, de préférence de 70/30 à 82/18, plus préférentiellement de 75/25 à 80/20.

La fraction enrichie en globulines et la fraction enrichie en albumines thermisée sont mélangées en milieu humide et ledit mélange est ensuite séché. Le mélange en milieu humide peut notamment être réalisé dans tout récipient convenant à cet effet, de préférence équipé d'un système d'agitation adéquat tel qu'un arbre mobile équipé de pales ou de turbines. Après obtention d'un mélange homogène, celui-ci est séché à l'aide de techniques bien connues de l'homme du métier telles que l'atomisation, préférentiellement l'atomisation dite « multiple-effet », ou bien la lyophilisation.

Le procédé selon l'invention peut en outre comprendre une ou plusieurs étapes facultatives avant et/ou après l'une des étapes a) b) c) d) ou e). Selon un mode de réalisation particulier, le procédé selon l'invention peut comprendre une étape d'hydrolyse enzymatique de la fraction protéique enrichie en globulines et/ou de la fraction protéique enrichie en albumines traitée entre l'étape c) et l'étape e). Le type d'enzyme utilisé dans la réaction d'hydrolyse enzymatique est de préférence une enzyme du groupe des protéases.

Sans être liée par une quelconque théorie, la demanderesse s'est aperçue que c'est le choix

- d' une fraction riche en albumines particulière en ce que son activité émulsifiante est supérieur à 600 ml d'huile par gramme de protéines, préférentiellement supérieur à 800 ml d'huile par gramme de protéines, encore plus préférentiellement supérieur à 1000 ml d'huile par gramme de protéines
- puis de son mélange par voie humide avec une fraction riche en globuline suivi d'un séchage de la solution ainsi obtenue, préférentiellement par atomisation

qui permet d'obtenir les propriétés uniques de la composition sujette de la présente invention.

La présente invention a également pour objet l'utilisation de la composition de protéines de pois selon l'invention dans une composition alimentaire ou pharmaceutique.

En effet, du fait de son excellente qualité nutritionnelle et de sa faible allergénicité, une telle composition de protéines de pois est d'un intérêt certain dans de nombreuses applications industrielles, en particulier dans l'industrie agroalimentaire ou pharmaceutique, et en nutrition animale.

Par composition alimentaire on entend une composition destinée à l'alimentation humaine ou animale. Le terme composition alimentaire englobe les produits alimentaires et les compléments alimentaires. Par composition pharmaceutique on entend une composition destinée à un usage thérapeutique.

Les exemples qui suivent permettent de mieux illustrer la demande, sans toutefois en limiter la portée.

**Exemple 1 : Production de farine de pois et des fractions enrichies respectivement en globulines et en albumines de pois** .

De la farine de pois est initialement préparée par broyage de pois fourragers décortiqués sur broyeur à marteaux de type ALPINE équipé d'une grille de 100 $\mu$m. Cette farine sera nommée « farine de pois ».

300 kg de farine de pois à 87% en poids de matière sèche sont ensuite mis à tremper dans de l'eau à la concentration finale de 25% en poids de matière sèche (MS), à un pH de 6,5. 1044 kg de suspension de farine à 25% en poids de MS (soit donc 261 kg de farine sèche) sont alors introduits avec 500 kg d'eau dans une batterie d'hydrocyclones composée de 14 étages. Elle est alimentée par la suspension de farine à l'étage n° 5. Cette séparation conduit à l'obtention d'une phase légère qui correspond à la sortie de l'étage n° 1. Elle est constituée d'un mélange de protéines, de fibres et de solubles. Cette phase légère en sortie d'hydrocyclones renferme en mélange (142 kg de MS au total) : les fibres (environ 14,8% en poids, soit 21 kg de MS), les protéines (environ 42,8% en poids, soit 60,8 kg de MS) et les solubles (environ 42,4% en poids, soit 60,2 kg de MS). Cette fraction présente une teneur en MS de 11,4% en poids. On procède à la séparation des fibres sur décanteurs centrifuges de type WESTFALIA employés dans une unité industrielle féculière de traitement de la pomme de terre. La phase légère en sortie de décanteur centrifuge renferme un mélange de protéines et de solubles, tandis que la phase lourde renferme les fibres de pois. La phase lourde renferme 105 kg de fibres à 20% en poids de MS. On constate que la quasi-totalité des fibres est bien retrouvée dans cette fraction. Quant à la fraction protéines et matières solubles, elle renferme 1142 kg d'un mélange en solution de matières solubles et de protéines (fraction à 6% en poids de MS). On procède à la floculation des protéines à leur point isoélectrique par ajustement de la phase légère en sortie de décanteur centrifuge à un pH de 4,5 et chauffage à 50°C. Les protéines ainsi mises à floculer sont laissées 10 minutes en cuve de maturation. Après précipitation des protéines, on procède à une décantation centrifuge, qui permet de récupérer du sédiment renfermant 56 kg de protéines (86% de N×6,25 sur sec) à 20% en poids de MS et un surnageant contenant entre autre la fraction protéique contenant les albumines. Ce sédiment correspond à la fraction protéique enrichie en globulines et il sera nommé « fraction enrichie en globulines ». Le surnageant correspond à la fraction protéique enrichie en albumines et il sera nommé « fraction enrichie en albumines ».

On procède ensuite au raffinage de la fraction enrichie en albumines. Son pH est ajusté à 7.0 par ajout de soude à 50%. La température de la suspension ainsi obtenue a été amenée à 70°C. La solution est pompée au travers d'une unité de microfiltration équipée de membranes céramiques type Inside Ceram® ayant un seuil de coupure de 0,14 $\mu$m (19 canaux de 4,5 mm). Tout au long de la filtration, la température est régulée à 60°C et la pression transmembranaire maintenue à une valeur comprise entre 0,4 et 0,6 bar. 707 litres de perméat de microfiltration et 1768 litres de rétentat de microfiltration sont ainsi récupérés. 550 litres du perméat sont pompés au travers d'une unité d'ultrafiltration. L'unité d'ultrafiltration est équipée de membranes céramiques type KERASEP® BX commercialisées par la société NOVASEP et ayant un seuil de coupure de 15 kDa (7 canaux de 6 mm). Tout au long de la filtration, la température est régulée à 60°C et la pression transmembranaire maintenue à une valeur comprise entre 1 et 3 bars. 467 litres de perméat d'ultrafiltration et 33 litres de rétentat contenant 75% en poids de protéines à 7,2% en poids de MS sont ainsi récupérés. Ce rétentat d'ultrafiltration correspond à la fraction protéique enrichie en albumines traitée et sera nommé « fraction enrichie en albumines traitée ».

Le pH de la fraction enrichie en albumines traitée est alors rectifié sous agitation à pH 6,8 par ajout de soude concentrée à 50%. Un traitement thermique UHT est ensuite appliqué en faisant passer la fraction enrichie en albumines traitée sur un skid VOMATEC, à une température de 140°C pendant un temps de contact d'une dizaine de secondes puis en flashant sous vide à environ 90°C. Le produit final sera nommé « fraction enrichie en albumines thermisée ».

**Exemple 2 : Préparation de compositions de protéines de pois selon l'invention**

On utilise une cuve en acier inoxydable équipée d'un agitateur motorisé. On introduit dans cette cuve 1,89 kg de « fraction enrichie en globulines » et 2 kg de « fraction enrichie en albumines thermisée ». Ce mélange permet l'obtention d'un ratio exprimé en pourcentage relatif de matière sèche entre la « fraction enrichie en globulines » et la « fraction enrichie en albumines thermisée » de 75/25. On démarre ensuite l'agitateur motorisé et on homogénéise le produit pendant 15 à 30 minutes. Le mélange est ensuite envoyé vers une tour d'atomisation simple effet afin d'être séché. On récupère ainsi une poudre titrant 95% en poids de MS. Le produit sera nommé « composition protéique de pois 75/25 ».

On utilise à nouveau la cuve précédente en acier inoxydable équipée d'un agitateur motorisé. On introduit dans cette cuve 2,5 kg de « fraction enrichie en globulines » et 2 kg de « fraction enrichie en albumines thermisée ». Ce mélange permet l'obtention d'un ratio exprimé en pourcentage relatif de matière sèche entre la « fraction enrichie en globulines » et la « fraction enrichie en albumines thermisée » de 80/20. On démarre ensuite l'agitateur motorisé et on homogénéise le produit pendant 15 à 30 minutes. Le mélange est ensuite envoyé vers une tour d'atomisation simple effet afin d'être séché. On récupère ainsi une poudre titrant 96% en poids de MS. Le produit sera nommé « composition protéique de pois 80/20 ».

**Exemple 2 bis: Préparation de compositions de protéines de pois hors invention, utilisant des « fraction enrichie en albumines traitée »**

On utilise une cuve en acier inoxydable équipée d'un agitateur motorisé. On introduit dans cette cuve 1,89 kg de « fraction enrichie en globulines » et 2 kg de « fraction enrichie en albumines traitée ». Comme décrit ci-dessus dans l'exemple 2, cette fraction n'est pas neutralisée à 6,8 et ne subit pas de traitement UHT. Ce mélange permet l'obtention d'un ratio exprimé en pourcentage relatif de matière sèche entre la « fraction enrichie en globulines » et la « fraction enrichie en albumines traitée » de 75/25. On démarre ensuite l'agitateur motorisé et on homogénéise le produit pendant 15 à 30 minutes. Le mélange est ensuite envoyé vers une tour d'atomisation simple effet afin d'être séché. On récupère ainsi une poudre titrant 95% en poids de MS. Le produit sera nommé « composition protéique de pois 75/25 ».

**Exemple 3 : Méthodologie du calcul de la digestibilité et du PDCAAS**

La mesure de la digestibilité protéique chez le rat est décrite dans l'article de la FAO suivant : « Protein Quality Evaluation. Report of a Joint FAO/WHO Expert Consultation. Rome, Italy."

Pour cela, l'aliment expérimental est composé de 10% en poids des protéines à tester, 1% en poids d'un mélange de vitamines AIN93, 3,5% en poids d'un mélange de minéraux AIN93, 0,2% en poids de bitartrate de choline, 5% en poids de cellulose, 10% en poids d'huile de maïs. L'aliment est complété à 100% avec de l'amidon de maïs.

Ce même aliment ne contenant pas de protéine sera utilisé comme témoin de l'essai. Pour cela, les protéines seront substituées par de l'amidon de maïs.

Des rats Sprague-Dawley en croissance (poids compris entre 50 et 70g au début de l'essai) seront hébergés individuellement dans des cages à métabolisme à une température comprise entre 18 et 24°C et une humidité comprise entre 40 et 70%. Les rats seront nourris avec un aliment standard au minimum 2 jours avant le début de l'essai. Ils sont ensuite nourris avec les régimes expérimentaux pendant une durée minimum de 9 jours constituée d'une première période d'acclimatation aux régimes de 4 jours suivie par une période de 5 jours de recueil des fèces. L'eau est donnée ad-libitum pendant toute la durée de l'étude. Les fèces ainsi recueillies quotidiennement sont pesées, lyophilisées pendant 24 heures et broyées. La mesure de l'azote contenu dans l'aliment et dans les fèces permettra de calculer la digestibilité protéique. La méthode de mesure utilisée est la méthode Kjeldahl.

L'azote ingéré et l'azote excrété est obtenu en multipliant la prise alimentaire ou le poids des fèces par les taux d'azote respectifs. Le taux d'azote basal est obtenu par la mesure de l'azote fécal des animaux nourris par le régime ne contenant pas de protéines.

La digestibilité protéique est obtenue de la façon suivante :

```
Digestibilité = [Azote ingéré-(Azote fécal-Azote basal)]/Azote
ingéré *100
```

L'aminogramme ou profil en acides aminés totaux est établi à l'aide de la méthode officielle NF EN ISO13903 :2005.

Le score en acides aminés est déterminé comme étant l'acide aminé essentiel limitant par rapport au profil de référence déterminé chez l'adulte. Pour l'obtenir, il faut calculer le ratio suivant : [mg de l'acide aminé contenu dans 1g de la protéine test]/[mg de l'acide aminé du profil de référence]. La plus petite valeur représente le score en acides aminés.

Le PDCAAS (Protein digestibility corrected amino acid score) est obtenu est multipliant ce score en acide aminé limitant par la digestibilité protéique déterminée chez le rat.

Le profil de référence chez l'adulte est décrit par la FAO dans son article : « Protein and amino acid requirements in human nutrition. Report of a joint WHO/FAO/UNU expert consultation. Geneva, Switzerland. 2007.

| Acide aminé essentiel | g/g de protéine |
|---|---|
| Histidine | 15 |
| Isoleucine | 30 |
| Leucine | 59 |
| Lysine | 45 |
| Méthionine | 16 |

(suite)

| Acide aminé essentiel | g/g de protéine |
|---|---|
| Cystéine | 6 |
| Méthionine + Cystéine | 22 |
| Phénylalanine + Tyrosine | 30 |
| Thréonine | 23 |
| Tryptophane | 6 |
| Valine | 39 |

**Exemple 4 : Méthodologie de mesure de la teneur en facteurs anti-trypsiques**

La méthode de mesure de la teneur en facteurs anti-trypsiques consiste à extraire par de la soude les inhibiteurs de la trypsine. Des volumes croissants de l'échantillon dilué sont mis en contact avec un excès de trypsine en présence de N-alpha-benzoyl D L-arginine p-nitroanilide (BAPNA) qui va alors être hydrolyse sous forme de p-nitroaniline, composé absorbant à 410 nm. Après blocage de la réaction à l'acide acétique, l'augmentation de coloration est mesurée au spectrophotomètre à 410 nm. La teneur en inhibiteurs est alors calculée à partir de la vitesse de diminution de la coloration. Une unité de trypsine est arbitrairement définie comme la quantité d'enzyme nécessaire pour entraîner un accroissement de 0,01 unités de l'absorbance à 410 nm pour 10 ml de mélange réactionnel dans les conditions de la méthode AOCS Ba 12-75. La teneur en facteur anti-trypsiques s'exprime en unité d'inhibition de la trypsine par mg d'échantillon à tester (UIT/mg).

**Exemple 5 : Comparaison des différentes fractions**

Le tableau ci-dessous synthétise les analyses du PDCAAS (selon exemple 3), de la teneur en facteurs anti-trypsiques (selon exemple 4) et de la capacité émulsifiante (selon méthode explicitée dans lea description).

| Référence | Teneur en facteurs anti-trypsiques (UIT/mg) | Digestibilité | PDCAAS | Capacité émulsifiante (en ml d'huile par g de protéines) |
|---|---|---|---|---|
| Farine de pois | Pas réalisé | 77,3 | 0,63 | |
| Fraction enrichie en globulines | 3 | 97,3 | 0,93 | |
| Fraction enrichie en albumines traitée | 41 | 96,9 | 0,56 | 500 |
| Fraction enrichie en albumines thermisée | 9,8 | 97,1 | Pas réalisé | 1300 |
| Composition protéique de pois 75/25 selon l'invention | 4 | 97,0 | 1 | 400 |
| Composition protéique de pois 80/20 selon l'invention<br>**compositions de protéines de pois hors invention, utilisant des « fraction enrichie en albumines traitée »** | 5 | 7,2 | 1 | 450<br><br>100 |

Ces exemples démontrent bien la concentration des facteurs anti-trypsiques dans la fraction enrichie en albumines, ce qui confirme donc l'enseignement général du domaine technique indiquant que ces fractions de bas poids moléculaire et hydrosolubles sont riches en facteurs anti-trypsiques. L'homme de l'art aurait donc été dissuadé de réutiliser cette fraction dans l'intention d'améliorer le PDCAAS de la fraction enrichie en globulines. Il aurait plutôt choisi d'utiliser des sources complémentaires telles que des protéines de blé comme enseigné dans l'art antérieur. La Demanderesse a été au-delà de cet enseignement et a développé une solution permettant l'obtention d'une protéine dont le PDCAAS est égal à 1 uniquement basé sur des fractions protéiques issues du pois.

Exemple 8 : Réalisation de boisson type « Ready To Drink » ou « Prête à Boire »

Les différentes compositions sont comparés par leur utilisation dans la formulation de boissons dites « prêtes à boire » ou « Ready To Drink ». Les différents composants sont résumés dans le tableau suivant :

| | Contrôle 100% lait | Contrôle Isolat classique | Invention 1 | Invention 2 | Hors invention |
|---|---|---|---|---|---|
| eau | 60 | | | | |
| Maltodextrine Glucidex IT19 (ROQUETTE) | 18,74 | 18,34 | 18,51 | 18,51 | 18,51 |
| Protéine de lait MPI Prodiet 85b | 10,8 | 5,53 | 5,53 | 5,53 | 5,53 |
| Isolat de pois Nutralys S85F | 0 | 5,67 | 0 | 0 | 0 |
| Composition protéique de pois 75/25 selon l'invention | 0 | 0 | 5,5 | 0 | 0 |
| Composition protéique de pois 80/20 selon l'invention | 0 | 0 | 0 | 5,5 | 0 |
| Composition de protéines de pois hors invention, utilisant des « fraction enrichie en albumines traitée » | 0 | 0 | 0 | 0 | 5,5 |
| huile de colza | 3,78 | | | | |
| saccharose | 3,4 | | | | |
| huile de tournesol | 2,52 | | | | |
| lecithine de soja | 0,4 | | | | |
| arôme vanille | 0,36 | | | | |

Le procédé de production des boissons est le suivant :

• Mélange à sec des poudres (protéines, maltodextrines and saccharose),

• Chauffer l'eau à 50°C, ajout des poudres, dispersion avec un agitateur Silverson pendant 30 min_à 50°C_3500 tr/min, ajout arôme vanille,

• Mélange et fonte séparée de la lécithine de soja et de l'huile à 50°C,

• APrés 30 min, ajout du mélange léctihine/huile à la solution aqueuse, mélange sous haute agitation 5min à 10000tr/min,

• Chauffage à 75°C

• Homogenisation à 200 bars_en 2 étapes

• Refroidissement et stockage à 4°C.

Afin de quantifier la qualité d'émulsion dans les boissons, on mesure la taille des particules à l'aide d'un Particle Size Analyser 3000 de la société MALVERN. Le Dmode représente la taille moyenne des particules émulsifiées.

| en microns | Contrôle 100% lait | Contrôle Isolat classique | Invention 1 | Invention 2 | Hors invention |
|---|---|---|---|---|---|
| D10 | 0,186 | 0,565 | 0,203 | | 3,61 |

(suite)

| en microns | Contrôle 100% lait | Contrôle Isolat classique | Invention 1 | Invention 2 | Hors invention |
|---|---|---|---|---|---|
| D50 | 0,546 | 43,1 | 0,482 | | 9,34 |
| D90 | 1,66 | 105 | 6,2 | | 103 |
| D mode | 0,577 | 69,1 | 0,444 | | 6,19 |
| D 4,3 | 3,48 | 47,9 | 3,08 | | 35,1 |

Seules les boissons réalisées avec l'invention permettent l'obtention de particules aussi bien émulsionnées qu'avec le témoin 100% lait référence.

**Revendications**

1. Composition de protéines de pois comprenant des globulines et des albumines **caractérisée en ce que** l'extrait sec de la composition :

   - comprend au moins 80% en poids, de préférences de 80 à 99% en poids, plus préférentiellement de 85 à 98% en poids, de 90 à 97% en poids, de 92 à 95% en poids de protéines par rapport au poids de l'extrait sec ;
   - présente un ratio massique des globulines sur les albumines de 65/35 à 85/15, de préférence de 70/30 à 82/18, plus préférentiellement de 75/25 à 80/20,
   les albumines présentant une activité émulsifiante supérieure à 600 mL d'huile de maïs par gramme d'albumines, et l'extrait sec de la composition présente une teneur en facteurs anti-trypsiques de 1 à 5 UIT/mg.

2. Composition selon la revendication 1, **caractérisée en ce que** les albumines présentent une activité émulsifiante supérieure à 800, de préférence supérieure à 1000 mL d'huile de maïs par gramme d'albumines.

3. Composition selon l'une quelconque des revendications 1 ou 2, **caractérisée en ce qu'**elle présente un PDCAAS égal à 1.

4. Procédé de préparation d'une composition de protéines de pois selon l'une des revendications précédentes comprenant les étapes suivantes :

   a) extraire les globulines et les albumines du pois pour obtenir une fraction protéique ;
   b) séparer les globulines des albumines pour obtenir une fraction enrichie en globulines et une fraction enrichie en albumines ;
   c) réduire la teneur en facteurs anti-trypsiques de la fraction enrichie en albumines pour obtenir une fraction enrichie en albumines traitée avec un procédé comprenant les étapes suivantes :

   c-i) faire une microfiltration ou une centrifugation de la fraction enrichie en albumines pour obtenir un perméat de microfiltration ou un surnageant de centrifugation; et
   c-ii) faire une ultrafiltration dudit perméat de microfiltration ou dudit surnageant de centrifugation pour obtenir un rétentat d'ultrafiltration, correspondant à la fraction enrichie en albumines traitée;

   d) ajuster le pH à une valeur comprise entre 6,5 et 7,5 puis chauffer la fraction enrichie en albumines traitée à une température comprise entre 130°C et 150°C, préférentiellement 140°C, pendant une durée comprise entre 5 et 15 secondes, préférentiellement 10 secondes pour obtenir une fraction enrichie en albumines thermisée dont l'activité émulsifiante est supérieure à 600 mL d'huile de maïs par gramme de protéines ;
   e) mélanger en présence d'eau la fraction enrichie en globulines et la fraction enrichie en albumines thermisée de manière à ce que l'extrait sec du mélange présente un ratio massique des globulines sur les albumines de 65/35 à 85/15, de préférence de 70/30 à 82/18, plus préférentiellement de 75/25 à 80/20 ;
   f) sécher la solution ainsi obtenue.

5. Procédé selon la revendication 4 **caractérisé en ce que** dans l'étape b) les globulines sont séparées des albumines avec un procédé comprenant les étapes suivantes :

b-i) faire floculer des globulines de la fraction protéique pour obtenir une phase solide C en suspension dans une phase liquide D ; et

b-ii) séparer la phase liquide D, contenant les albumines, de la phase solide C, contenant les globulines.

6. Procédé selon l'une des revendications 4 ou 5 **caractérisé en ce que** dans l'étape c) la fraction enrichie en albumines traitée présente une teneur en facteurs anti-trypsiques dans l'extrait sec de 20 à 80 UIT/mg, de préférence de 30 à 50 UIT/mg.

7. Utilisation de la composition de protéines de pois telle que définie à l'une quelconque des revendications 1 à 3 dans une composition alimentaire ou pharmaceutique.

**Patentansprüche**

1. Erbsenproteinzusammensetzung, die Globuline und Albumine umfasst, **dadurch gekennzeichnet, dass** der Trockenextrakt der Zusammensetzung:

   - mindestens 80 Gew.-%, vorzugsweise 80 bis 99 Gew.-%, besonders bevorzugt 85 bis 98 Gew.-%, 90 bis 97 Gew.-%, 92 bis 95 Gew.-% Proteine, bezogen auf das Gewicht des Trockenextrakts, enthält;
   - ein Massenverhältnis von Globulinen zu Albuminen von 65/35 bis 85/15, vorzugsweise von 70/30 bis 82/18, besonders bevorzugt von 75/25 bis 80/20 aufweist,
   wobei die Albumine eine Emulgieraktivität von mehr als 600 mL Maisöl pro Gramm Albumine aufweisen,
   und der Trockenextrakt der Zusammensetzung einen Gehalt an antitryptischen Faktoren von 1 bis 5 ITU/mg aufweist.

2. Zusammensetzung nach Anspruch 1, **dadurch gekennzeichnet, dass** die Albumine eine Emulgieraktivität von mehr als 800, vorzugsweise mehr als 1000 mL Maisöl pro Gramm Albumine aufweisen.

3. Zusammensetzung nach einem der Ansprüche 1 oder 2, **dadurch gekennzeichnet, dass** sie einen PDCAAS von 1 aufweist.

4. Verfahren zur Herstellung einer Erbsenproteinzusammensetzung nach einem der vorhergehenden Ansprüche, umfassend die folgenden Schritte:

   a) Extrahieren der Globuline und Albumine aus der Erbse, um eine Proteinfraktion zu erhalten;
   b) Abtrennen der Globuline von den Albuminen, um eine mit Globulinen angereicherte Fraktion und eine mit Albuminen angereicherte Fraktion zu erhalten;
   c) Reduzieren des Gehalts an antitryptischen Faktoren in der mit Albuminen angereicherten Fraktion, um eine mit Albuminen angereicherte Fraktion zu erhalten, die mit einem Verfahren behandelt wurde, das die folgenden Schritte umfasst:

   c-i) Mikrofiltration oder Zentrifugation der mit Albuminen angereicherten Fraktion, um ein Mikrofiltrationspermeat oder einen Zentrifugationsüberstand zu erhalten; und
   c-iii) Ultrafiltration des Mikrofiltrationspermeats oder des Zentrifugationsüberstands, um ein Ultrafiltrationsretentat zu erhalten, das der behandelten, mit Albuminen angereicherten Fraktion entspricht;

   d) Einstellen des pH-Werts auf einen Wert zwischen einschließlich 6,5 und 7,5 und dann Erhitzen der behandelten, mit Albuminen angereicherten Fraktion auf eine Temperatur zwischen einschließlich 130°C und 150°C, vorzugsweise 140°C, für eine Dauer zwischen einschließlich 5 und 15 Sekunden, vorzugsweise 10 Sekunden, um eine wärmebehandelte, mit Albuminen angereicherte Fraktion zu erhalten, deren Emulgieraktivität mehr als 600 mL Maisöl pro Gramm Protein beträgt;
   e) Mischen der mit Globulinen angereicherten Fraktion und der wärmebehandelten, mit Albuminen angereicherten Fraktion in Gegenwart von Wasser, so dass der Trockenextrakt der Mischung ein Massenverhältnis von Globulinen zu Albuminen von 65/35 bis 85/15 aufweist, vorzugsweise von 70/30 bis 82/18, besonders bevorzugt von 75/25 bis 80/20;
   f) Trocknen der so erhaltenen Lösung.

5. Verfahren nach Anspruch 4, **dadurch gekennzeichnet, dass** in Schritt b) die Globuline von den Albuminen mit einem Verfahren abgetrennt werden, das die folgenden Schritte umfasst:

b-i) Ausflocken von Globulinen aus der Proteinfraktion, um eine feste Phase C zu erhalten, die in einer flüssigen Phase D suspendiert ist; und

b-iii) Abtrennen der flüssigen Phase D, welche die Albumine enthält, von der festen Phase C, welche die Globuline enthält.

6. Verfahren nach einem der Ansprüche 4 oder 5, **dadurch gekennzeichnet, dass** in Schritt c) die behandelte, mit Albuminen angereicherte Fraktion einen Gehalt an antitryptischen Faktoren im Trockenextrakt von 20 bis 80 ITU/mg aufweist, vorzugsweise von 30 bis 50 ITU/mg.

7. Verwendung der Erbsenproteinzusammensetzung, wie in einem der Ansprüche 1 bis 3 definiert, in einer Lebensmittelzusammensetzung oder pharmazeutischen Zusammensetzung.

**Claims**

1. A pea protein composition comprising globulins and albumins, **characterized in that** the solids extract of the composition:

- comprises at least 80% by weight, preferably from 80 to 99% by weight, more preferentially from 85 to 98% by weight, from 90 to 97% by weight, from 92 to 95% by weight of proteins relative to the weight of the solids extract;
- has a mass ratio of globulins to albumins from 65/35 to 85/15, preferably from 70/30 to 82/18, more preferentially from 75/25 to 80/20,
the albumins having an emulsifying activity greater than 600 ml of corn oil per gram of albumins,
and the solids extract of the composition having a content of antitryptic factors of 1 to 5 TIU/mg.

2. The composition as claimed in claim 1, **characterized in that** the albumins have an emulsifying activity greater than 800, preferably greater than 1000 ml of corn oil per gram of albumins.

3. The composition as claimed in any one of claims 1 or 2, **characterized in that** it has a PDCAAS equal to 1.

4. A process for producing a pea protein composition as claimed in any one of the previous claims comprising the following steps:

a) extracting the globulins and albumins from pea in order to obtain a protein fraction;
b) separating the globulins from the albumins in order to obtain a globulin-enriched fraction and an albumin-enriched fraction;
c) reducing the content of antitryptic factors in the albumin-enriched fraction in order to obtain an albumin-enriched fraction treated with a process comprising the following steps:

c-i) carrying out a microfiltration or a centrifugation of the albumin-enriched fraction in order to obtain a microfiltration permeate or a centrifugation supernatant; and
c-ii) carrying out an ultrafiltration of said microfiltration permeate or of said centrifugation supernatant in order to obtain an ultrafiltration retentate, corresponding to the treated albumin-enriched fraction;

d) adjusting the pH to a value between 6.5 and 7.5, then heating the treated albumin-enriched fraction at a temperature between 130°C and 150°C, preferably 140°C, for a period of between 5 and 15 seconds, preferentially 10 seconds, in order to obtain a thermized albumin-enriched fraction having an emulsifying activity greater than 600 ml of corn oil per gram of albumins;
e) mixing, in the presence of water, the globulin-enriched fraction and the thermized albumin-enriched fraction so that the solids extract of the mixture has a mass ratio of globulins to albumins from 65/35 to 85/15, preferably 70/30 to 82/18, more preferentially from 75/25 to 80/20;
f) drying the solution thus obtained.

5. The process as claimed in claim 4, **characterized in that**, in step b), the globulins are separated from the albumins with a process comprising the following steps:

b-i) flocculating globulins of the protein fraction in order to obtain a solid phase C suspended in a liquid phase D; and

b-ii) separating the liquid phase D, containing the albumins, from the solid phase C, containing the globulins.

6. The process as claimed in any one of claims 4 or 5, **characterized in that**, in step c,) the treated albumin-enriched fraction has a content of antitryptic factors in the solids extract of 20 to 80 TIU/mg, preferably 30 to 50 TIU/mg.

7. The use of the pea protein composition as defined in any one of claims 1 to 3, in a food or pharmaceutical composition.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2012047252 A
- WO 2007017572 A

- EP 1400537 A

**Littérature non-brevet citée dans la description**

- *Pea protein*
- *Food Research International.*, 2015, vol. 76, 160-167
- *Roquette Nutralys*
- *J. Sci. Food Agric.*, 1990, vol. 53, 107-110
- **C.LEFRANC-MILLOT.** *Vegetable Protein : A winner ?*, 2014

- **SHERMAN, P.** A critique of some methods proposed for evaluating the emulsifying capacity and emulsion stabilizing performance of vegetable proteins. *Ital. J. Food Sci.*, 1995, vol. 1, 3
- *Protein and amino acid requirements in human nutrition. Report of a joint WHO/FAO/UNU expert consultation. Geneva, Switzerland,* 2007